# EUROPÄISCHE PATENTSCHRIFT

(11) **EP 1 478 793 B1**
(45) Veröffentlichungstag und Bekanntmachung des Hinweises auf die Patenterteilung: **09.01.2008**
(21) Anmeldenummer: 03744340.5
(22) Anmeldetag: 05.03.2003
(51) Int. Cl.: C30B 7/00, B01L 3/02, C12N 5/00

(54) **VORRICHTUNG ZUR ERZEUGUNG VON HÄNGENDEN TROPFEN UND ZUGEHÖRIGES VERFAHREN**
DEVICE FOR CREATING HANGING DROPS AND CORRESPONDING METHOD
DISPOSITIF DE CRÉATION DES GOUETTES PENDANTES ET PROCÉDÉ CORRESPONDANT

(30) Priorität: 05.03.2002 DE 10210908
(43) Veröffentlichungstag der Anmeldung: 24.11.2004
(73) Patentinhaber: Kammer, Dr., Winfried, 72108 Rottenburg (DE)
(72) Erfinder: Kammer, Dr., Winfried, 72108 Rottenburg (DE)
(74) Vertreter: Patentanwälte Ruff, Wilhelm, Beier, Dauster & Partner
(86) Internationale Anmeldenummer: PCT/EP2003/002209
(87) Internationale Veröffentlichungsnummer: WO 2003/078700

(56) Entgegenhaltungen:
- EP-A- 0 608 423
- WO-A-00/00678
- WO-A-00/29114
- WO-A-00/44498
- WO-A-01/58593
- WO-A-99/00657
- DE-A- 19 843 234
- US-A- 3 682 323
- US-A- 4 687 529
- US-A- 5 143 659
- US-A- 5 221 410
- US-A- 5 792 653
- US-A- 5 958 345
- US-B1- 6 303 387
- LUFT J R ET AL: "EXPERIENCES WITH HANGMAN: A MACROMOLECULAR HANGING DROP VAPOR DIFUSION TECHNIQUE" JOURNAL OF CRYSTAL GROWTH, NORTH-HOLLAND PUBLISHING CO. AMSTERDAM, NL, Bd. 122, Nr. 1 / 4, 2. August 1992 (1992-08-02), Seiten 181-185, XP000306493 ISSN: 0022-0248

## Beschreibung

Die Erfindung betrifft eine Vorrichtung zur Erzeugung vor hängender Tropfen sowie ein Verfahren zur Erzeugung geeigneter Reaktions- und/oder Kultivierungsbedingungen für Zellen.

In der modernen Medizintechnik bzw. Biotechnologie und verwandten Bereichen besteht ein großer Bedarf an Vorrichtungen, mit deren Hilfe schnell, effizient und nach Möglichkeit mit einem geringen präparativen Aufwand Substanzen bzw. Substanzkombinationen auf z. B. ihre fruchtschädigende Eigenschaften, Toxizität (Toxizitätsprofil), Wirksamkeit, wirksame Konzentration etc., hin untersucht werden können. So wird z. B. bei einer Chemotherapie häufig vorher abgeklärt, ob die Chemostatika die Tumore abtöten können. Dies wird meistens in der Zellkultur getestet. Dabei werden Tumorzellen vom Patienten entnommen und kultiviert. Nachdem man eine ausreichende Dichte an Tumorzellen herangezüchtet hat, werden diese dann verschiedenen Chemostatika ausgesetzt. Diejenigen Chemostatika, die in der Zellkultur die besten Ergebnisse erzielen, d. h. die in der geringsten Konzentration die meisten Krebszellen abgetötet haben, werden dann bei der anschließenden Chemotherapie eingesetzt. Es stellt sich aber häufig heraus, daß die *in vitro* erzielten Ergebnisse *in vivo* nicht reproduzierbar sind. Das liegt unter anderem daran, daß die Bedingungen, die in einem Organismus herrschen, kaum in der Zellkultur imitiert werden können.

Schwierigkeiten macht auch die Untersuchung von Substanzen auf ihre fruchtschädigenden Eigenschaften. Ein Standardverfahren zur Durchführung solcher Untersuchungen ist die Inkubation von sogenannten embryoiden Körperchen. Embryoide Körperchen entstehen aus embryonalen Stammzellen durch Zell-Aggregation und nachfolgende Zell-Differenzierung. Aus den Zellen embryoider Körperchen können mittels Inkubation unter geeigneten Kultivierungsbedingungen Zellen definierten Differenzierungsstatus gezüchtet werden (z.B. neuronale Zellen, Myozyten oder Blutstammzellen). Dabei ist es bis heute nicht gelungen, embryoide Körperchen in einer Form zur Aufzucht zu bringen, bei der sie a) zuverlässig (homogen und automatisierbar) angesät, b) in Volumina von z.B. 40 µl und größer sicher gehandhabt (Robotik) und c) hochparallelisiert (automatisiert) analysiert werden können. Somit stehen bisher nur einzeln herangezüchtete embryoide Körperchen für dieses Standardverfahren zur Verfügung. Die Probleme bei der Kultivierung von embryoiden Körperchen liegen unter anderem darin, daß embryonale Stammzellen nur unter bestimmten Bedingungen zu embryoiden Körperchen aggregieren. So spielt z. B. die Konzentration an embryonalen Stammzellen sowie die Versorgung mit geeigneten Nährstoffen eine entscheidende Rolle. Außerdem darf den Zellen keine Möglichkeit gegeben werden, sich an eine Oberfläche anzuheften. Da nur mit hohem finanziellen und präparativen Aufwand einzeln kultivierte embryoide Körperchen für die Untersuchung auf fruchtschädigende Eigenschaften bereitgestellt werden können, und da die Kultivierungs- bzw. die Reaktionsbedingungen bei jedem Test stark variieren, sind die erhaltenen Ergebnisse untereinander nicht verläßlich vergleichbar.

Eine Reihe von Firmen hat daher Vorrichtungen bereitgestellt, die eine Lösung für diese Probleme versprechen. So hat z. B. die Firma MWG AG einen sogenannten Affimetrix Arrayer auf den Markt gebracht, der für die automatisierte Herstellung von DNA-Arrays genutzt werden soll. Dieser Affimetrix Arrayer macht sich die sogenannte Pin-and-Ring™-Technologie zunutze. Bei dieser Technologie wird ein sogenannter Pin (Metalldorn) durch eine Flüssigkeitsmembran durchgeführt, die von einem Ring gehalten wird. Dies ähnelt dem "Pustering" für die Erzeugung von Seifenblasen. Sobald der Pin durch die Flüssigkeitsmembran gestoßen wird, kann dann eine bestimmte Flüssigkeitsmenge mit diesem Pin auf das Array geladen werden. Dieses System kann aber nur zur Übertragung von geringen Flüssigkeitsmengen genutzt werden.

Dies gilt gleichermassen für die in der US 6 303 387 beschriebene Vorrichtung, die für den Transfer mikroskopischer Flüssigkeitsmengen, insbesondere zu Analysezwecken, vorgesehen ist.

Eine andere Firma, die Berkeley Lab Inc., stellt sogenannte Mikrokristallisationsroboter her. Diese Vorrichtungen kommen insbesondere dann zum Einsatz, wenn die dreidimensionale Struktur von Proteinen mit Hilfe der Kristallographietechnik untersucht werden soll. Um die Funktion eines Proteins zu verstehen, muß dessen Tertiärstruktur aufgeklärt werden. Dazu bedient man sich häufig der Kristallstrukturanalyse. Bei der Kristallstrukturanalyse wird die räumliche Anordnung der Atome in kristallinen Festkörpern mit Hilfe von Röntgen-, Elektronen- oder Neutronenstrahlen, deren Wellenlängen etwa den Atomabständen in Kristallgittern entsprechen, ermittelt. Bei der Ermittlung der Tertiärstruktur von Proteinen wird diese Technik als "protein-x-ray-cristallography" bezeichnet. Bei der Erzeugung der Kristalle kommt es aber häufig aufgrund unterschiedlicher Umgebungsbedingungen zu keinem gleichmäßigen Aufbau, was als Kristallbaufehler bezeichnet wird und zu falschen Ergebnissen bei der Kristallstrukturanalyse führen kann.

Eine weitere Möglichkeit eine Vielzahl von Untersuchungen gleichzeitig durchzuführen, stellen die Mikrotiterplatten dar. Bei den Mikrotiterplatten werden sowohl die (flüssigen) Medien als auch die zu untersuchenden Substanzen direkt in sogenannte Kavitäten eingefüllt, die letztendlich Reaktionskammern entsprechen, mit einem Boden und seitlichen Begrenzungen. In diesen Kammern findet dann die eigentliche Reaktion/Kultivierung statt.

Weitere Vorrichtungen zum Aufbringen von Medien sind auf dem Markt oder werden in den Forschungsabteilungen bzw. an den Universitäten getestet. Alle weisen aber diverse Nachteile auf. So ist bei dem schon beschriebenen Verfahren zur Untersuchung von Substanzen auf ihre fruchtschädigenden Eigenschaften ein Problem, daß die embryonalen Stammzellen nicht kontrolliert reproduzierbar zu homogenen embryoiden Körperchen aggregieren. Beim Ausplattieren auf z. B. einer Mikrotiterplatte wachsen die Stammzellen auf der gesamten Bodenfläche der Mikrotiterplatte an, wodurch eine Aggregation zu embryoiden Körperchen unterbleibt. Auch die schon beschriebenen Vorrichtungen sind nicht geeignet, geeignetere Kultivierungs-/Reaktionsbedingungen bereitzustellen. Es wird daher im kleinen Maßstab versucht, durch Auftragen der Flüssigkeit (bei Stammzellen das Kultivierungsmedium) auf z. B. einem Objektträger, welcher im Anschluß gedreht wird, einen sogenannten "hängenden Tropfen" herzustellen.

Solche "hängende Tropfen" haben einige Vorteile. So werden zum einen die untersuchten Substanzen vollständig von der Flüssigkeit umspült, was eine ausreichende Versorgung mit den benötigten Faktoren, wie z. B. lonen, Salzen, Differenzierungsfaktoren, Toxinen etc. erlaubt. Zum anderen erlauben "hängende Tropfen" die Aggregation von embryonalen Stammzellen zu embryoiden Körperchen, da die Zellen im Tropfen nach unten sinken, dort aber keinen Kontakt zu einer festen Oberfläche finden. Mangels anderer Anhaftungsstellen aggregieren die embryonalen Stammzellen und bilden embryoide Körperchen. Die Oberflächenspannung der Tropfen verhindert das Austreten von embryonalen Stammzellen und embryoiden Körperchen aus den Tropfen. Die Übertragung dieses Prinzips auf größere Maßstäbe zur Erzeugung geeigneter Kultivierungs-/Reaktionsbedingungen für standardisierte Untersuchung(en), wie sie z. B. bei der Evaluierung von Pharmazeutika benötigt werden, scheiterte aber bislang daran, daß für Probleme bezüglich der parallelisierten Handhabung solcher "hängender Tropfen" keine Lösungen gefunden wurden. So besteht beim Aufbringen mehrerer Tropfen auf eine Oberfläche immer die Gefahr, daß Tropfen ineinanderlaufen. Weiterhin können nur vergleichsweise geringe Volumina aufgebracht werden, da bei Überschreiten eines kritischen Volumens Tropfen bei Bewegung des Trägers unkontrollierbar werden. Schon bei einem Tropfenvolumen von 20 µl können sich Tropfen auf hydrophoben Untergründen bei der Handhabung deutlich bewegen. Um die Herstellung von homogenen hängenden Tropfen zu automatisieren, ist eine einfache und reproduzierbare Möglichkeit ein sogenanntes Toploading. Dabei wird ein bestimmtes Flüssigkeitsvolumen auf eine Unterlage von oben aufgebracht. Wird die Unterlage umgedreht, hängt der Tropfen nach unten. Beim Umdrehen können die Tropfen - abhängig von ihrer Größe - ihren Aufbringungsort verlassen. Es gilt aber die Regel, daß je größer der Tropfen ist, desto unkontrollierbarer ist er in der Handhabung. Andererseits erlauben größere Tropfen eine verlängerte Kultivierdauer ohne Mediumwechsel (das führt z.B. zu länger andauernden Differenzierungsprozessen), eine größere aufbringbare Zellmenge usw.. Außerdem wirken sich Verdunstungsprozesse bei größeren Tropfen nicht so stark aus wie bei kleineren, da das Oberfläche/Volumen-Verhältnis deutlich günstiger ist, als bei kleineren Tropfen. Dadurch wird die Reproduzierbarkeit der ablaufenden Vorgänge im Tropfen deutlich erhöht.

Daraus ergibt sich, daß die Tropfen als eigentliche Reaktionsgefäße
- möglichst groß sein sollten,
- reproduzierbar aufbringbar sein sollten,
- hochparallelisiert/automatisiert erzeugt und eingesetzt werden sollten.

Erwünscht ist daher eine Vorrichtung sowie ein Verfahren, mit deren Hilfe flüssige Medien aufgebracht werden können. Im Sinne der obigen Ausführungen sollen zum einen die aufbringbaren Volumina der Medien groß genug sein, um auch längerandauernde komplexe (bio)chemische und/oder biologische Reaktionen ablaufen zu lassen. Zum anderen sollen die Ergebnisse reproduzierbar sein.

Aufgabe der Erfindung ist es daher, eine Vorrichtung sowie ein Verfahren bereitzustellen, die universell zur Erzeugung solch geeigneter Bedingungen genutzt werden können und die beschriebenen Nachteile des Standes der Technik überwinden helfen. Ferner ist es Ziel dieser Erfindung, eine Vorrichtung sowie ein Verfahren bereitzustellen, die mit vergleichsweise geringem präparativen Aufwand die Erzeugung einer Vielzahl von (gleichen) Kultivierungs-/Reaktionsbedingungen ermöglichen.

Diese Aufgabe wird gelöst durch die Vorrichtung bzw. das Verfahren mit den Merkmalen der unabhängigen Ansprüche 1, 17 und 23. Bevorzugte Ausführungsformen sind in den abhängigen Ansprüchen dargelegt. Der Wortlaut sämtlicher Ansprüche wird hiermit durch Bezugnahme zum Inhalt dieser Beschreibung gemacht.

Überraschenderweise wurde gefunden, daß durch Anbringung von scharfkantigen Begrenzungen eine Stabilisierung von flüssigen Medien erfolgt, so daß auch bei größeren Volumina durch Drehen "hängende Tropfen" entstehen und die Tropfen nicht von der Oberfläche abreißen. Darüberhinaus kann hierdurch auch ein Verschieben der Tropfen vermieden werden.

Gegenstand der Erfindung ist somit eine Vorrichtung gemäß Anspruch 1. Unter planarer Erhebung soll dabei jede Erhebung mit im wesentlichen ebener oberer Begrenzungsfläche verstanden werden. Vorzugsweise ist die Erhebung dabei würfel- oder quaderförmig. Auch pyramidenstumpfförmige oder ähnliche Erhebungen sind möglich. Die Erhebung kann an ihrer oberen Begrenzungsfläche auch eine leicht konvexe oder konkave Ausgestaltung haben. Wesentlich ist, daß die auf die aus einem hydrophoben Trägermaterial bestehende Erhebung aufgebrachten flüssigen Medien durch die scharfkantige Begrenzung(en)/Abgrenzung(en) der oberen Begrenzungsfläche als Tropfen fixiert werden.

Gemäß einer bevorzugten Ausführungsform beträgt die Abmessung einer planaren Erhebung in Längsrichtung zwischen etwa 2 und etwa 7 mm, vorzugsweise zwischen etwa 3 und etwa 5 mm. In einer weiteren bevorzugten Ausführungsform beträgt die Abmessung einer planaren Erhebung in Querrichtung zwischen etwa 3 und etwa 9 mm, vorzugsweise zwischen etwa 4 und etwa 7 mm. In einem Ausführungsbeispiel weisen die Erhebungen eine Grösse von etwa 5 x 7 mm auf, in einem weiteren Beispiel eine Grösse von etwa 3 x 4 mm. Auch andere Abmessungen der Erhebung werden erfindungsgemäß beansprucht, sofern die Erhebung mindestens eine scharfkantige Begrenzung zur Stabilisierung des flüssigen Mediums aufweist. Wenn die Abmessungen der Erhebungen relativ klein sind, kann das vorteilhafterweise bewirken, dass hierdurch auch das Tropfenvolumen verkleinert wird und die Tropfen dadurch an Stabilität gewinnen.

In einer besonders bevorzugten Ausführungsform der erfindungsgemäßen Vorrichtung sind die planaren Erhebungen als schmale, langgestreckte Erhebungen ausgebildet. Auf derartige langgestreckte Erhebungen kann vorteilhafterweise ein Flüssigkeitsband aufgebracht werden. In einem solchen Flüssigkeitsband können beispielsweise im Bereich des Tissue Engineering langgestreckte Präparate kultiviert werden, wie z.B. Gefäße, insbesondere Blutgefäße oder Fasern, insbesondere Nervenfasern oder Muskelfasern. Die Abmessungen solcher langgestreckten Erhebungen hängen selbstverständlich vom jeweiligen Anwendungsfall ab. Die Länge kann sich von einigen mm, z.B. etwa 2 oder etwa 3 mm, bis über die gesamte Länge der erfindungsgemäßen Vorrichtung erstrecken, also über mehrere cm, z.B. bis zu 13 cm. Die Breite hängt ebenfalls vom Anwendungsfall ab und kann beispielsweise zwischen etwa 1 und etwa 5 mm betragen.

In einer weiteren bevorzugten Ausführungsform sind die planaren Erhebungen zwischen 1 und 5 mm, vorzugsweise ca. 2 mm hoch. Die Höhe der Erhebung richtet sich dabei insbesondere nach dem verwendeten hydrophoben Trägermaterial sowie dem zweckdienlichen Verfahren zur Produktion geeigneter Vorrichtungen. So richtet sich die Höhe einer Erhebung z. B. bei Fräsung nach der Frästiefe sowie der Linienbreite, die je nach Ausgestaltung der Vorrichtung variieren kann.

In einer weiteren bevorzugten Ausführungsform der Erfindung kann die Anzahl der planaren Erhebungen in Längsrichtung mindestens 18 und in Querrichtung mindestens 9 betragen. In einer anderen bevorzugten Ausführungsform beträgt die Anzahl der planaren Erhebungen in Längsrichtung 12 und in Querrichtung 8. Diese Ausführungsform entspricht von der Anordnung und Anzahl der Erhebungen im wesentlichen einer üblichen 96well-Mikrotiterplatte, was für manche Anwendungen vorteilhaft sein kann. Die Anzahl der Erhebungen ist abhängig von den Gesamtabmessungen der Vorrichtung, dem Verwendungszweck (z. B. Zellkultur) etc.. Die Erhebungen, sind in einer weiteren bevorzugten Ausführungsform voneinander zwischen 1 und 4 mm, vorzugsweise ca. 2 mm entfernt. Bevorzugte Abstände der Erhebungen voneinander liegen auch zwischen 0,5 bis 1,5 mm.

In einer bevorzugten Ausführungsform ist die scharfkantige Begrenzung recht- bis spitzwinklig. Unter spitzwinklig wird erfindungsgemäß ein Winkel < 90° verstanden.

Grundsätzlich können bei der Erfindung die verschiedensten Materialien/Trägermaterialien eingesetzt werden. Insbesondere handelt es sich dabei um Kunststoffe, z.B. organische Polymere.

In einer besonders bevorzugten Ausführungsform besteht die Vorrichtung mindestens teilweise, vorzugsweise vollständig, aus mindestens einem transparenten Material/Trägermaterial. Hierfür kommen z. B. Polystyrol und/oder Plexiglas in Frage. Die Verwendung eines transparenten Trägermaterials hat den Vorteil, daß die Auswertung mit Hilfe geeigneter optischer Verfahren, z. B. Mikroskopie, Fluoreszenzmessung etc. direkt auf der Vorrichtung selbst erfolgen kann.

In einer weiteren bevorzugten Ausführungsform ist die Vorrichtung insgesamt zwischen 10 und 30 mm, vorzugsweise zwischen 10 und 23 mm, hoch. In Längsrichtung können die Abmessungen der Vorrichtung zwischen 100 und 150 mm, vorzugsweise ca. 130 mm, und in Querrichtung zwischen 80 und 100 mm, vorzugsweise ca. 90 mm, betragen, was in einer weiteren bevorzugten Ausführungsform beansprucht wird. Die Abmessungen der Vorrichtung können sich dabei nach dem erwünschten Einsatzort richten. So wird z. B. die Vorrichtung bei Einsatz in einem Zellinkubator den Abmessungen in dessen Innenraum entsprechend angepaßt werden. Soll die erfindungsgemäße Vorrichtung in z. B. ein Rundgefäß eingepaßt werden, so entsprechen die maximalen Längs- bzw. Querabmessungen der Vorrichtung dem Durchmesser des Rundgefäßes.

Die Vorrichtung hat in einer weiteren bevorzugten Ausführungsform zum Angreifen mindestens eine, vorzugsweise zwei Haltemöglichkeiten, so daß die Handhabung und der Transport der Vorrichtung erleichtert wird. Bei den Haltemöglichkeiten handelt es sich vorzugsweise um insbesondere zwei Haltegriffe, die an insbesondere gegenüberliegenden äußeren Kanten der Vorrichtung angebracht sind. Diese können beispielsweise als einfache Vorsprünge an den Kanten ausgebildet sein.

In einer weiteren bevorzugten Ausführungsform der erfindungsgemäßen Vorrichtung ist diese Vorrichtung derart ausgebildet, dass sie auf einem Untergrund, insbesondere einem Trägergestell, abgestellt werden kann. Vorzugsweise ist es hierbei vorgesehen, dass die Vorrichtung in beiden Orientierungen abstellbar ist derart, dass im abgestellten Zustand einmal die mit Proben zu beladende Seite nach oben weisen kann, was eine Beladung sehr einfach macht. Im anderen Fall weist dann die Oberfläche, die für die Beladung vorgesehen ist, nach unten, so dass dies beispielsweise die Position für eine Kultivierung oder Reaktion im hängenden Tropfen wäre. Vorteilhafterweise können für diesen Zweck seitliche Vorsprünge an zwei Außenkanten der Vorrichtung vorgesehen sein, mit denen die Vorrichtung zum einen transportiert werden kann und zum anderen auf einem entsprechenden Untergrund/Gestell in der jeweils gewünschten Orientierung abgestellt werden kann.

In einer weiteren bevorzugten Ausführungsform der erfindungsgemäßen Vorrichtung hat diese mindestens eine, vorzugsweise mehrere Standflächen, insbesondere Standfüße. Wird z. B. die erfindungsgemäße Vorrichtung freistehend benutzt, dann kann die Vorrichtung vier Standfüße aufweisen, um einen festen Stand zu haben.

In einer besonders bevorzugten Ausführungsform sind mindestens zwei Vorrichtungen miteinander bzw. aneinander lösbar befestigbar. So ist es z. B. erfindungsgemäß möglich, mehrere Vorrichtungen durch Rast-, Steck- oder Klemmverschlüsse miteinander zu verbinden. Dabei können die Vorrichtungen nebeneinander und/oder übereinander (aufeinander gestapelt) angeordnet sein. Diese miteinander verbundenen und/oder aufeinander gestapelten Vorrichtungen können dann beispielsweise gemeinsam transportiert und/oder inkubiert - z. B. in der Zellkultur - werden. Alternativ können Einschubvorrichtungen für ein bis mehrere Vorrichtungen für Transport und Inkubation benutzt werden.

Gemäß der Vorliegender Erfindung sind zwischen 10 und 80 µl, vorzugsweise 40 bis 80 µl, flüssiges Medium je Erhebung aufbringbar.

Die erfindungsgemäße Vorrichtung ist nach Aufbringen der flüssigen Medien um mindestens 90°, vorzugsweise ca. 180°, drehbar, was in einer weiteren Ausführungsform beansprucht wird. Durch Drehen der Vorrichtung werden dann sogenannte "hängende Tropfen" erzeugt.

Ein entsprechendes Gestell zur Ablage der beschriebenen Vorrichtung wird gleichfalls von der Erfindung mit umfasst. Dieses ist vorzugsweise so ausgebildet, dass zwei bis vier in etwa rechtwinklig aufeinanderstoßende, und aneinander befestigte Holme einen Rahmen als Unterlage bilden, auf den die erfindungsgemäße Vorrichtung, die an mindestens zwei Kanten Vorsprünge aufweist, aufgelegt wird. An dem so gebildeten Trägergestell können an den Oberkanten an entsprechender Stelle Aussparungen vorgesehen sein, um den Vorsprüngen der aufzulegenden Vorrichtung einen sicheren Halt zu gewähren. Die Vorsprünge an der aufzulegenden Vorrichtung, die vorteilhafterweise auch als Tragegriffe eingesetzt werden können, sowie auch die entsprechenden Aussparungen im Trägergestell finden sich vorzugsweise an gegenüberliegenden Seiten der aufzulegenden Vorrichtung bzw. des Trägergestells. In einer besonders bevorzugten Ausführungsform weist das Trägergestell drei Holme auf, die sozusagen ein offenes Rechteck bilden. Es kann jedoch auch bevorzugt sein, dass das Trägergestell aus zwei in etwa rechtem Winkel aufeinanderstoßende und aneinander befestigten Holmen besteht. Weiterhin kann es auch bevorzugt sein, dass das Trägergestell von vier Holmen gebildet wird, die sozusagen ein geschlossenes Rechteck bilden.

Weiterhin wird ein Verfahren zur Erzeugung geeigneter Reaktions-und/oder Kultivierungsbedingungen gemäß Anspruch 17 beansprucht, in dem eine erfindungsgemäße Vorrichtung benutzt wird. Bei den Kultivierungsbedingungen kann es sich um geeignete Bedingungen für Proliferations- und/oder Differenzierungsassays für eukaryotische Zellen handeln. Bei den eukaryotischen Zellen handelt es sich insbesondere um menschliche oder tierische Zellen bzw. um Zellverbände, Gewebe, Organoide oder Organe,

Das erfindungsgemäße Verfahren kann weiterhin dadurch gekennzeichnet sein, dass es sich bei den Kultivierungsbedingungen um Wachstums- und/oder Differenzierungsbedingungen für eukaryotische Zellen zum Zweck der Gewebekultur und insbesondere zum Zweck des Tissue Engineerings handelt. Weiterhin kann das Verfahren vorteilhaft in der Weise eingesetzt werden, dass es sich bei den Kultivierungsbedingungen um fördernde und/oder hemmende und/oder abtötende Bedirigungen für die Kultivierung von Tumorzellen und/oder Tumorgeweben handelt. Mit Hilfe eines solchen Verfahrens können beispielsweise Reagenzien getestet werden, die in einer Chemotherapie eingesetzt werden sollen. In diesem künstlichen System kann vor der eigentlichen Therapie untersucht werden, ob bestimmte Chemotherapeutika für bestimmte Tumorzellen und/oder -gewebe den gewünschten Effekt aufweisen oder nicht.

Weiterhin kann das erfindungsgemäße Verfahren so ausgestaltet sein, dass es sich bei den Kultivierungsbedingungen um Wachstums-und/oder Differenzierungsbedingungen für Zellaggregate und/oder Gewebe zur Beeinflussung angiogenetischer Prozesse in diesen Aggregaten oder Geweben handelt. Hiermit könnten beispielsweise verschiedene Faktoren untersucht und getestet werden, die derartige angiogenetische Prozesse, die auch bei der Tumorentwicklung entscheidend beteiligt sein können, fördern oder hemmen.

Weiterhin kann es sich in einer weiteren bevorzugten Ausführungsform bei den Reaktionsbedingungen um geeignete Kristallisierungs- und/oder Röntgenstrukturanalysebedingungen handeln. Die erfindungsgemäße Vorrichtung kann genutzt werden, um z. B. Kristallisierungskammern in Tropfenform zu erzeugen. Nach Kristallisierung der Proteine kann so z. B. mit Hilfe von Röntgen-, Elektronen- oder Neutronenstrahlen die dreidimensionale Struktur der Proteine festgestellt werden.

Ferner umfasst die Erfindung ein Verfahren zur Nutzung von Tropfen als Reaktionsstätte gemäß Anspruch 23 wobei ein flüssiges Medium, beispielsweise ein Kulturmedium für die Zellkultur, auf eine oben beschriebene Vorrichtung aufgebracht (beladen) wird und die Vorrichtung anschließend mit der beladenen Seite nach unten ausgerichtet wird. Hierbei kann sich die Unterseite im wesentlichen horizontal oder aber auch in einem Winkel von beispielsweise 90° oder weniger zur Unterlage befinden. Die so gebildete Reaktionsstätte kann beispielsweise für die Kultivierung von biologischem Material, insbesondere von eukaryotischen Zellen, Zellaggregaten und/oder Zellgeweben genutzt werden. Andererseits ist es auch mit Vorteil möglich, derartige Reaktionsstätten für Kristallisierungsprozesse im Hinblick auf Strukturanalysen zu nutzen. Des weiteren umfasst die Erfindung selbstverständlich auch andere Einsatzmöglichkeiten, bei welchen ein Tropfen als Reaktionsstätte nutzbar ist.

Zum Abnehmen der Tropfen von der erfindungsgemäßen Vorrichtung kann die Vorrichtung mit der beladenen Seite nach unten einer im wesentlichen planaren Oberfläche genähert werden, so dass sich die Tropfen auf dieser Oberfläche absetzen. Hierbei werden die Vorrichtung und die Oberfläche so nahe zusammengeführt, bis die Tropfen die Oberfläche berühren und so auf die Oberfläche übergehen. Geeignete Oberflächen können beispielsweise zumindest teilweise aus Glas und/oder Kunststoff bestehen.

In einer weiteren bevorzugten Ausführungsform dieses Aspektes der Erfindung wird das Abnehmen der Tropfen mit Hilfe von mindestens einem Abstandshalter durchgeführt, der sich auf der Oberfläche und/oder auf der Vorrichtung mit den Tropfen befinden. Hierdurch wird vermieden, dass die Proben in den Tropfen, beispielsweise Zellen oder Gewebe, durch Quetschungen beschädigt werden.

In einer anderen bevorzugten Ausführungsform dieses Verfahrens wird die Vorrichtung zum Abnehmen der Tropfen mit der beladenen Seite nach unten an mindestens eine Vertiefung in der Weise herangeführt, dass die Tropfen mit mindestens einer Seitenwand der Vertiefung in Kontakt treten und daran herablaufen. Vorteilhafterweise handelt es sich bei geeigneten Vertiefungen um die Vertiefungen einer Mikrotiterplatte oder ähnliches.

Weitere Einzelheiten und Merkmale der Erfindung ergeben sich aus der nachfolgenden Beschreibung der Darstellung von bevorzugten Ausführungsformen der erfindungsgemäßen Vorrichtung und des Verfahrens in Verbindung mit den Ansprüchen. Hierbei können die jeweiligen Merkmale für sich alleine oder zu mehreren in Kombination miteinander verwirklicht sein.

In den Abbildungen zeigen:
- Fig. 1:: Auf- und Seitenansicht einer möglichen Ausgestaltung der erfindungsgemäßen Vorrichtung.
- Fig. 2:: Aufsicht auf eine erfindungsgemäße Vorrichtung in unbeladenem Zustand.
- Fig.3:: Seitenansicht auf eine mit Flüssigkeit beladene erfindungsgemäße Vorrichtung.
- Fig. 4:: Aufsicht auf eine auf der Seite stehende unbeladene erfindungsgemäße Vorrichtung.
- Fig. 5:: Schrägansicht eines erfindungsgemäßen Trägergestells und einer erfindungsgemäßen Vorrichtung einzeln und in zusammengesetztem Zustand.
- Fig. 6:: Eine bevorzugte erfindungsgemäße Ausführungsform zum Abnehmen der Tropfen.
- Fig. 7:: Eine weitere bevorzugte erfindungsgemäße Ausführungsform zum Abnehmen der Tropfen.

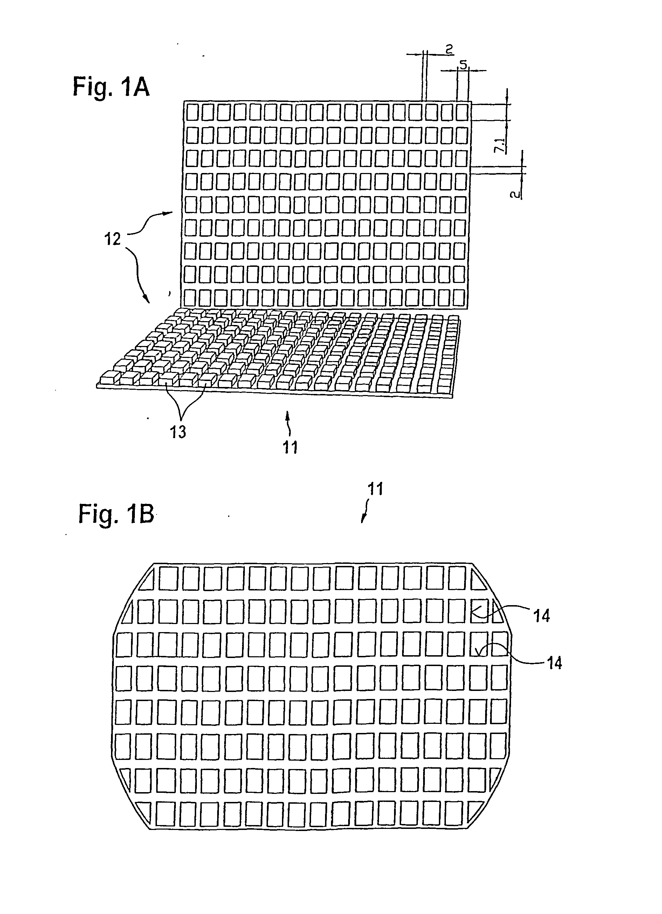

Fig. 1 zeigt eine schematische Darstellung einer möglichen Ausgestaltung der erfindungsgemäßen Vorrichtung (11). Die Vorrichtung (11) besteht aus einem Trägermaterial (12) und einer Vielzahl von Erhebungen (13), mit im wesentlichen planarer oberer Begrenzungsfläche. Weiterhin werden die scharfkantigen Begrenzungen (14) gezeigt. Die einzelnen (planaren) Erhebungen (13) sind voneinander jeweils ca. 2 mm entfernt. Die Abmessungen einer Erhebung betragen in Längsrichtung ca. 5 mm und in Querrichtung ca. 7,1 mm. Insgesamt sind in Längsrichtung 18 und in Querrichtung 9 Erhebungen dargestellt.

Fig. 1A zeigt eine Aufsicht und perspektivische Seitenansicht der Vorrichtung 11. Eine solche Vorrichtung kann z. B. aus einer Polystyrolplatte (ca. 4 mm x 130 mm x 130 mm) oder einem Plexiglasblock hergestellt werden. Dabei werden mittels einer Fräse die dargestellten Erhebungen herausgefräst, wobei linienartige Vertiefungen von ca. 2 mm Breite und ca. 2 mm Tiefe aus der Platte bzw. aus dem Block herausgefräst werden. Je nachdem, welche Abmessungen die Vorrichtung haben soll, kann diese danach bearbeitet werden. So können Ausstülpungen der Polystyrolplatte als Haltegriffe dienen. Auch können z. B. die Ecken der Vorrichtung (11) abgerundet werden, wie in Fig. 1B dargestellt, so daß diese in ein Rundgefäß mit entsprechendem Durchmesser eingesetzt werden kann. Ferner können auch Standfüße an der Vorrichtung angebracht werden, z. B. durch Ankleben, damit die Vorrichtung eine bessere Standfestigkeit hat.

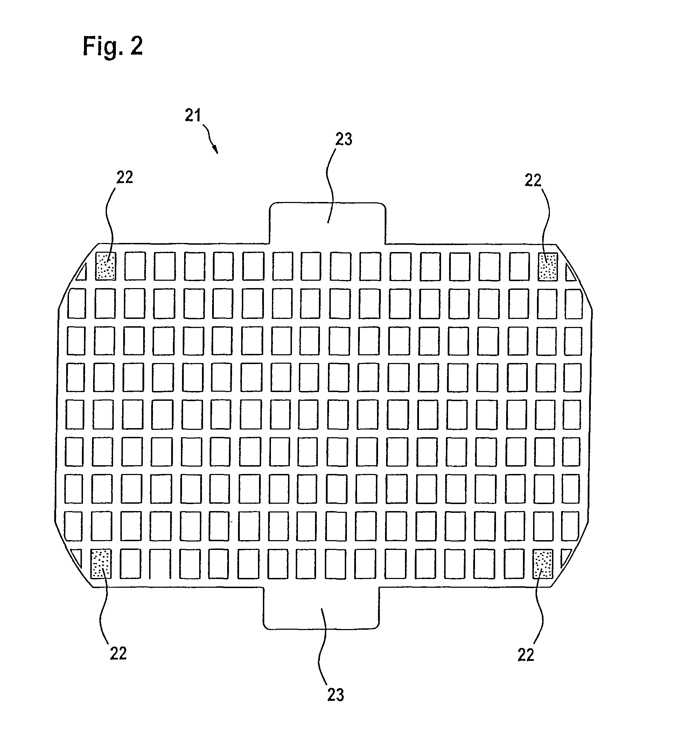

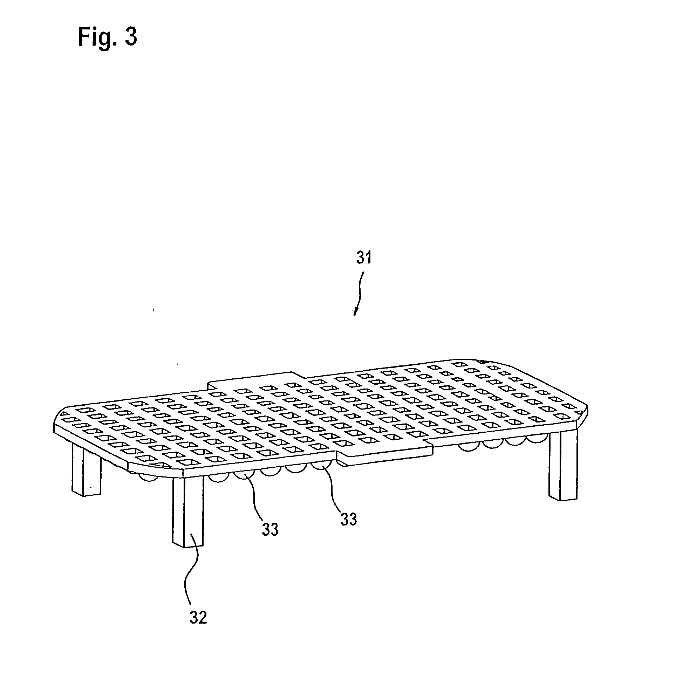

Fig. 2 zeigt eine Aufsicht auf eine erfindungsgemäße Vorrichtung. Diese Vorrichtung wurde wie bei Fig. 1 beschrieben hergestellt. Die als Platte ausgebildete Vorrichtung 21 steht auf vier Standfüßen 22 und befindet sich noch im unbeladenen Zustand. An den beiden Längsseiten der Vorrichtung 21 sind Vorsprünge 23 angebracht, die zum Transportieren und Abstellen der Vorrichtung auf einem entsprechenden Gestell genutzt werden können. Die Beladung der Platte erfolgt, indem auf jede Erhebung, die sich hier auf der vom Betrachter abgewandten Seite befindet, ein gewisses Volumen einer geeigneten Lösung, beispielsweise eines Kulturmediums, aufgebracht wird. Für die Beladung der erfindungsgemäßen Vorrichtung liegt diese vorteilhafterweise auf dem Rücken, d. h. Standfüße 22 würden senkrecht nach oben zeigen. Bereits beim Beladen bildet sich die im wesentlichen rechteckige, an den Ecken abgerundete Form der aufgebrachten Tropfen. Nach dem Aufstellen der beladenen Platte auf die Standfüße zeigen sich auf der vom Betrachter abgewandten Seite abgerundete, klare Abgrenzungen der tropfenförmig an jeder Erhebung anhaftenden Lösung an den Rändern (Kanten) jeder Erhebung. Die Tropfen würden in Blickrichtung nach unten hängen. Jeder einzelne dieser Tropfen wird dabei durch die scharfkantigen Abgrenzungen bzw. Kanten an den Rändern jeder Erhebung stabilisiert, so dass ein Abreißen oder Verschieben des Tropfens verhindert wird. Dies ist in Fig. 3 dargestellt.

Fig. 3 zeigt eine mit Kulturmedium beladene und in Nutzposition gebrachte Vorrichtung 31. Auch hierbei steht die Vorrichtung auf vier Standfüßen 32. Es ist eine schräge Seitenansicht dargestellt. Die Tropfen 33 sind an der Unterseite der Vorrichtung 31 zu erkennen.

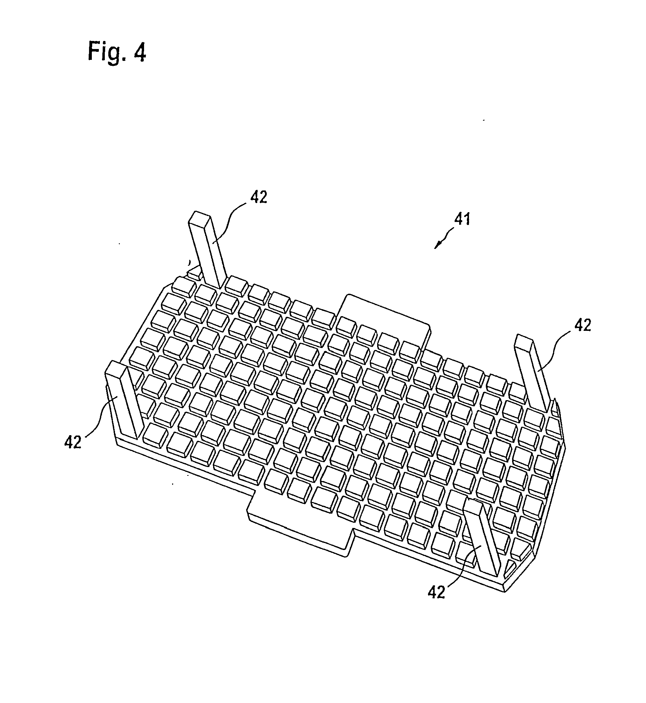

Fig. 4 zeigt eine angelehnte, auf der Seite stehende Vorrichtung 41. Die Standfüße 42 der Platte stehen dabei in etwa horizontal zum Untergrund, während die Platte im wesentlichen vertikal zum Untergrund ausgerichtet ist. Es ist die Vorrichtung im unbeladenen Zustand gezeigt. Diese Position kann bei entsprechender Schräge auch für die Kultivierung eingesetzt werden.

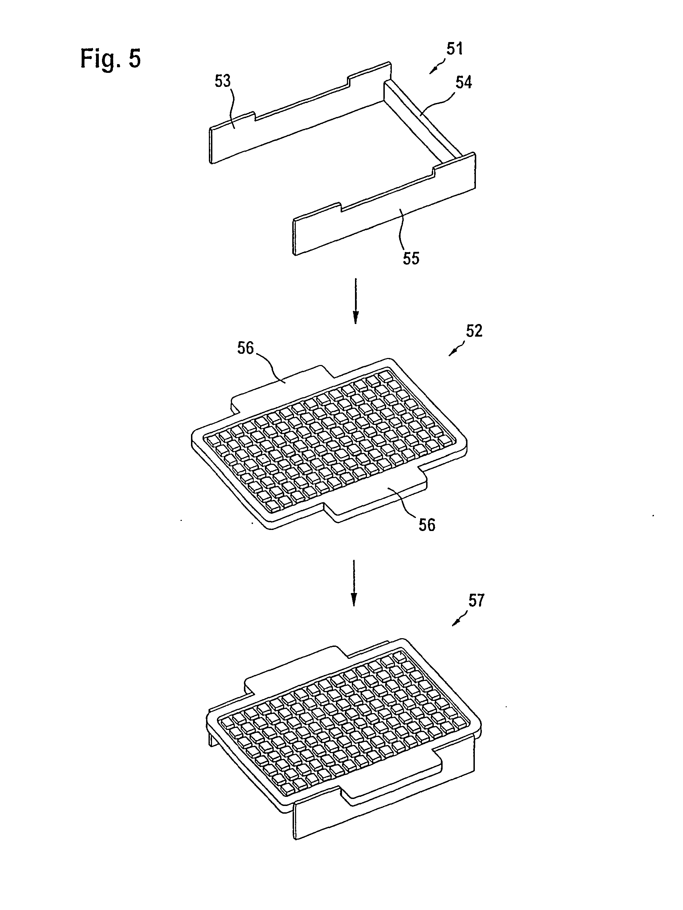

Fig. 5 zeigt oben ein Trägergestell 51, welches für das Abstellen einer erfindungsgemäßen Vorrichtung 52, wie sie in der Mitte gezeigt ist, geeignet ist. Das Trägergestell 51 besteht aus drei im rechten Winkel an den Schmalseiten aufeinanderstoßenden Holmen 53, 54, 55, die einen rechtwinkligen Rahmen bilden. An den oberen Kanten der zwei sich, gegenüberliegenden Holme 53 und 55 befindet sich jeweils eine Aussparung, die für die Aufnahme von Vorsprüngen 56 an zwei sich gegenüberliegenden Kanten der Vorrichtung 52 vorgesehen ist. Weiterhin ist die obere Kante der Stirnseite des Holmes 54 etwas niedriger als die oberen Kanten der Seitenholme 53 und 55, damit die Tropfen an der Vorrichtung 52 nicht mit dem stirnseitigen Holm 54 des Gestells 51 in Kontakt treten können. Hierdurch wird ein stabiler und sicherer Halt der erfindungsgemäßen Vorrichtung 52 auf dem Gestell 51 gewährleistet, wie dem unteren Teil der Figur 5 zu entnehmen ist. Die erfindungsgemäße Vorrichtung 52 kann entweder mit der zu beladenden oder der bereits beladenen Oberfläche nach oben oder nach unten auf das Trägergestell 51 aufgelegt werden. So kann das Trägergestell 51 zum Beladen oder Bearbeiten der Proben sowie auch zum beispielsweise Kultivieren in den hängenden Tropfen genutzt werden.

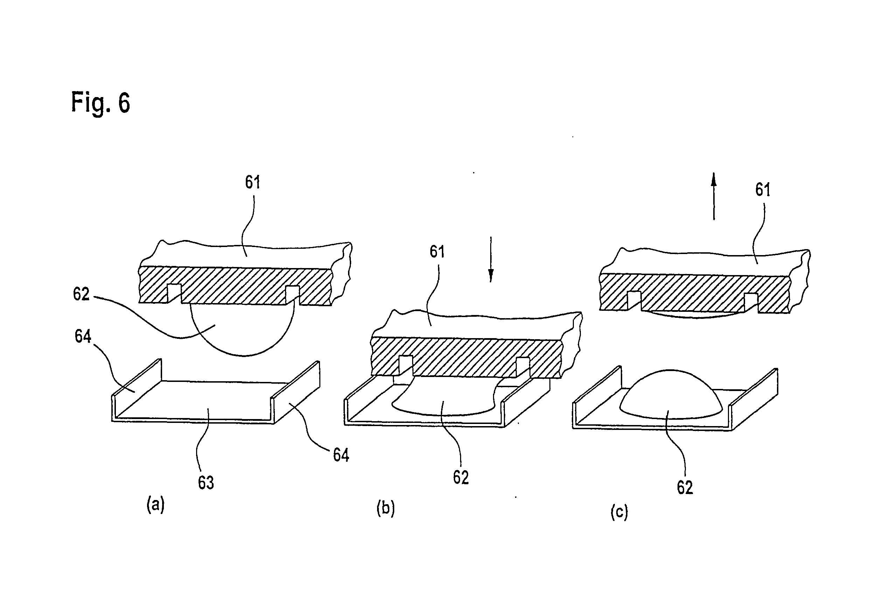

Aus Fig. 6 geht das Abnehmen der Tropfen von einer erfindungsgemäßen Vorrichtung hervor. Die erfindungsgemäße Vorrichtung 61 mit dem daranhängenden Tropfen 62 wird einer im wesentlichen planaren Oberfläche 63 zugeführt (a), bis der Tropfen 62 die Oberfläche 63 berührt und an der Oberfläche 63 abgesetzt werden kann (b). Nach dem Absetzen des Tropfens 62 auf der Oberfläche 63 wird die Vorrichtung 61 wieder entfernt (c). Fig. 6 zeigt gleichfalls zwei Abstandshalter 64 an der Oberfläche 63, die dafür sorgen, dass ein Mindestabstand zwischen der Vorrichtung 61 und der im wesentlichen planaren Oberfläche 63 eingehalten wird, so dass Quetschungen des Präparates im Tropfen 62 vermieden werden.

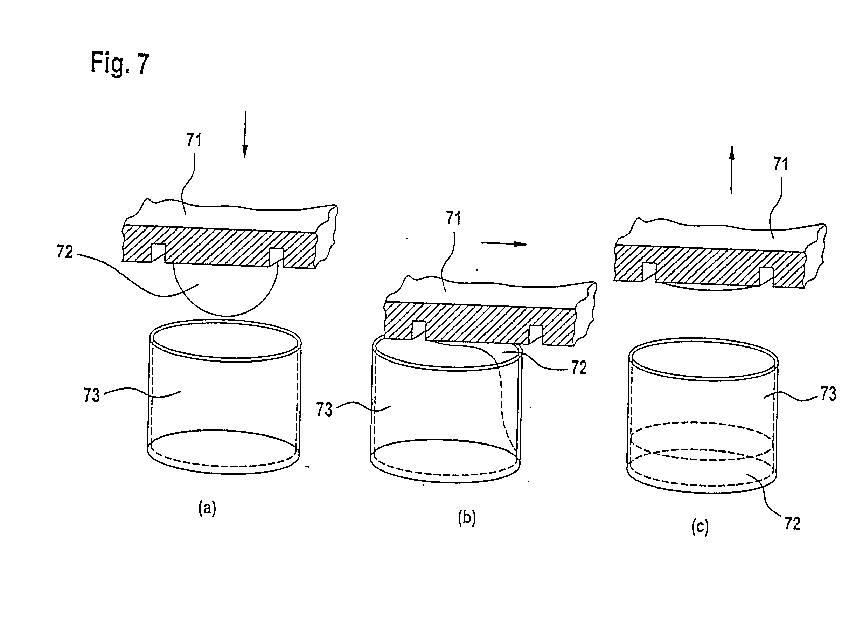

Fig. 7 zeigt eine weitere vorteilhafte Ausgestaltung des Abnehmens des Tropfens. Hierbei wird der sich an der erfindungsgemäßen Vorrichtung 71 befindende Tropfen 72 an eine Vertiefung 73 herangeführt (a). Die Vorrichtung 71 wird dann so verschoben, dass der Tropfen 72 eine Seitenwand der Vertiefung 73 berührt und an der Seitenwand herabläuft (b). Schließlich wird die Vorrichtung 71 von der Vertiefung 73 entfernt. Der Tropfen 72 befindet sich nun in der Vertiefung 73 (c). Vorteilhafterweise handelt es sich bei der Vertiefung 73 um die Kavität einer Mikrotiterplatte oder ähnliches. Selbstverständlich kann es sich bei der Vertiefung auch um ein separates Reaktionsgefäß handeln.

## Patentansprüche

1. Vorrichtung zur Erzeugung von hängenden Tropfen, wobei die Vorrichtung eine Platte mit mindestens zwei aus einem hydrophoben Material bestehenden Erhebungen mit im wesentlichen planarer rechteckförmiger oberer Begrenzungsfläche aufweist, auf die jeweils zwischen 10 und 80 µl flüssiges Medium aufbringbar sind, wobei der Abstand zwischen benachbarten Erhebungen mindestens 0,5 mm beträgt und die Erhebungen an der oberen planaren Begrenzungsfläche mindestens zwei parallel zueinander angeordnete scharfkantige Bagrenzungen aufweisen und die Abmessungen der Vorrichtung im wesentlichen denen einer Mikrotiterplatte entsprechen.

2. Vorrichtung nach Anspruch 1, **dadurch gekennzeichnet, daß** die Abmessungen der Erhebungen in der ersten Richtung zwischen 2 und 7 mm betragen.

3. Vorrichtung nach einem der Ansprüche 1 oder 2, **dadurch gekennzeichnet, daß** die Abmessungen der Erhebungen in der zweiten Richtung zwischen etwa 3 und etwa 9 mm betragen.

4. Vorrichtung nach einem der Ansprüche 1 bis 3, **dadurch gekennzeichnet, dass** die Erhebungen schmale, langgestreckte Erhebungen sind.

5. Vorrichtung nach einem der Ansprüche 1 bis 4; **dadurch gekennzeichnet, daß** die Erhebungen zwischen 1 und 5 mm hoch sind.

6. Vorrichtung nach einem der Ansprüche 1 bis 5, **dadurch gekennzeichnet, daß** die Anzahl der Erhebungen in Längsrichtung mindestens 18 und in Querrichtung mindestens 9 beträgt.

7. Vorrichtung nach einem der Ansprüche 1 bis 5, **dadurch gekennzeichnet, dass** die Anzahl der Erhebungen in Längsrichtung 12 und in Querrichtung 8 beträgt.

8. Vorrichtung nach einem der Ansprüche 1 bis 7, **dadurch gekennzeichnet, daß** der Abstand zwischen zwei benachbarten Erhebungen zwischen 0,5 und 4 mm beträgt.

9. Vorrichtung nach einem der Ansprüche 1 bis 8, **dadurch gekennzeichnet, daß** die Vorrichtung mindestens teilweise aus mindestens einem transparentem Material besteht.

10. Vorrichtung nach Anspruch 9, **dadurch gekennzeichnet, daß** das Material Polystyrol und/oder Plexiglas ist.

11. Vorrichtung nach einem der Ansprüche 1 bis 10, **dadurch gekennzeichnet, daß** die Abmessungen der Vorrichtung in der ersten Richtung zwischen 100 und 150 mm und in der zweiten Richtung zwischen 80 und 110 mm betragen.

12. Vorrichtung nach einem der Ansprüche 1 bis 11, **dadurch gekennzeichnet, daß** die Vorrichtung eine Gesamthöhe zwischen 10 und 30 mm aufweist.

13. Vorrichtung nach einem der Ansprüche 1 bis 12, **dadurch gekennzeichnet, daß** die Vorrichtung mindestens einen Haltegriff hat.

14. Vorrichtung nach einem der Ansprüche 1 bis 13, **dadurch gekennzeichnet, dass** die Vorrichtung auf einem Trägergestell abstellbar ausgebildet ist, wobei vorzugsweise die Vorrichtung mit der zu beladenden oder beladenen Oberfläche nach oben oder nach unten abstellbar ist.

15. Vorrichtung nach einem der Ansprüche 1 bis 14, **dadurch gekennzeichnet, daß** die Vorrichtung mindestens einen Standfuß hat.

16. Vorrichtung nach einem der Ansprüche 1 bis 15, **dadurch gekennzeichnet, daß** sie stapelbar ausgebildet ist.

17. Verfahren zur Erzeugung geeigneter Reaktions- und/oder Kultivierungsbedingungen für Zellen, ausgenommen humane embryonale Stammzellen, **gekennzeichnet durch** die Verwendung einer Vorrichtung gemäß einen der vorhergehender Ansprüche, wobei die Erhebungen mit einem flüssigen Medium beladen werden und **durch** Drehen der Vorrichtung hängende Tropfen erzeugt werden.

18. Verfahren nach Anspruch 17, **dadurch gekennzeichnet, daß** es sich bei den Kultivierungsbedingungen um Proliferations- und/oder Differenzierungsbedingungen für eukaryotische Zellen handelt.

19. Verfahren nach Anspruch 17 oder Anspruch 18, **dadurch gekennzeichnet, dass** das Verfahren zum Zweck der Gewebekultur und/oder des Tissue Engineering eingesetzt wird.

20. Verfahren nach einem der Ansprüche 17 bis 19, **dadurch gekennzeichnet, dass** es sich bei den Kultivierungsbedingungen um fördernde und/oder hemmende und/oder abtötende Bedingungen für die Kultivierung von Tumorzellen und/oder Tumorgeweben handelt.

21. Verfahren nach einem der Ansprüche 17 bis 20, **dadurch gekennzeichnet, dass** es sich bei den Kultivierungsbedingungen um Wachstums- und/oder Differenzierungsbedingungen für Zellaggregate und/oder Gewebe zur Beeinflussung angiogenetischer Prozesse in diesen Zellaggregaten und/oder Geweben handelt.

22. Verfahren nach Anspruch 17, **dadurch gekennzeichnet, daß** es sich bei den Reaktionsbedingungen um Kristallisierungs- und/oder Röntgenstrukturanalysebedingungen handelt.

23. Verfahren zur Nutzung von Tropfen als Reaktionsstätte, ausgenommen für humane embryonale Stammzellen, **dadurch gekennzeichnet, dass** ein flüssiges Medium auf eine Vorrichtung, gemäß einem der Ansprüche 1 bis 16, aufgebracht wird und die Vorrichtung mit der beladenen Seite nach unten im wesentlichen horizontal oder in einem Winkel von etwa 90° oder weniger zu einer Unterlage ausgerichtet wird.

24. Verfahren nach Anspruch 23, **dadurch gekennzeichnet, dass** zum Abnehmen des Tropfens die Vorrichtung mit der beladenen Seite nach unten einer im wesentlichen planaren Oberfläche derart zugeführt wird, dass sich der Tropfen auf der Oberfläche absetzt.

25. Verfahren nach Anspruch 24, **dadurch gekennzeichnet, dass** die Oberfläche mit mindestens einem Abstandshalter versehen ist.

26. Verfahren nach Anspruch 25, **dadurch gekennzeichnet, dass** zum Abnehmen des Tropfens die Vorrichtung mit der beladenen Seite nach unten an mindestens eine Vertiefung derart herangeführt wird, dass der Tropfen mindestens eine Seitenwand der Vertiefung berührt und daran herabläuft.

27. Verfahren nach Anspruch 26, **dadurch gekennzeichnet, dass** die Vertiefung eine Vertiefung einer Mikrotiterplatte ist.

## Claims

1. Device for creating hanging drops, the device having a plate with at least two elevations made of a hydrophobic material and with a substantially planar and rectangular upper limit surface onto which in each case between 10 and 80 µl of liquid medium can be applied, the distance between adjacent elevations being at least 0.5 mm, and the elevations on the upper planar limit surface having at least two sharp-edged boundaries arranged parallel to each other, and the dimensions of the device corresponding substantially to those of a microtitre plate.

2. Device according to Claim 1, **characterized in that** the dimensions of the elevations in the first direction are between 2 and 7 mm.

3. Device according to one of Claims 1 and 2,
**characterized in that** the dimensions of the elevations in the second direction are between approximately 3 and approximately 9 mm.

4. Device according to one of Claims 1 to 3,
**characterized in that** the elevations are narrow, elongate elevations.

5. Device according to one of Claims 1 to 4,
**characterized in that** the elevations are between 1 and 5 mm high.

6. Device according to one of Claims 1 to 5,
**characterized in that** the number of elevations is at least 18 in the longitudinal direction and at least 9 in the transverse direction.

7. Device according to one of Claims 1 to 5,
**characterized in that** the number of elevations is 12 in the longitudinal direction and 8 in the transverse direction.

8. Device according to one of Claims 1 to 7,
**characterized in that** the distance between two adjacent elevations is between 0.5 and 4 mm.

9. Device according to one of Claims 1 to 8,
**characterized in that** the device is made at least partially of at least one transparent material.

10. Device according to Claim 9, **characterized in that** the material is polystyrene and/or Plexiglas.

11. Device according to one of Claims 1 to 10,
**characterized in that** the dimensions of the device are between 100 and 150 mm in the first direction and between 80 and 110 mm in the second direction.

12. Device according to one of Claims 1 to 11,
**characterized in that** the device has an overall height of between 10 and 30 mm.

13. Device according to one of Claims 1 to 12,
**characterized in that** the device has at least one grip.

14. Device according to one of Claims 1 to 13,
**characterized in that** the device is designed such that it can be placed on a supporting frame, the device preferably being able to be placed thereon with the surface to be charged, or the charged surface, facing upwards or downwards.

15. Device according to one of Claims 1 to 14,
**characterized in that** the device has at least one stand.

16. Device according to one of Claims 1 to 15,
**characterized in that** it is designed to be stackable.

17. Method for creating suitable reaction and/or cultivation conditions for cells, excluding human embryonic stem cells, **characterized by** the use of a device according to one of the preceding claims, the elevations being charged with a liquid medium, and hanging drops being created by turning the device.

18. Method according to Claim 17, **characterized in that** the cultivation conditions are proliferation and/or differentiation conditions for eukaryotic cells.

19. Method according to Claim 17 or Claim 18,
**characterized in that** the method is used for the purpose of tissue culture and/or tissue engineering.

20. Method according to one of Claims 17 to 19,
**characterized in that** the cultivation conditions are stimulating and/or inhibiting and/or destroying conditions for the cultivation of tumour cells and/or tumour tissues.

21. Method according to one of Claims 17 to 20,
**characterized in that** the cultivation conditions are growth and/or differentiation conditions for cell aggregates and/or tissues for influencing angiogenic processes in these cell aggregates and/or tissues.

22. Method according to Claim 17, **characterized in that** the reaction conditions are crystallization and/or X-ray structure analysis conditions.

23. Method for using drops as a reaction site, except for human embryonic stem cells, **characterized in that** a liquid medium is applied to a device according to one of Claims 1 to 16, and the device, with the charged side facing downwards, is oriented substantially horizontally or at an angle of about 90° or less with respect to a base.

24. Method according to Claim 23, **characterized in that**, to remove the drop, the device, with the charged side facing downwards, is brought towards a substantially planar surface in such a way that the drop settles on the surface.

25. Method according to Claim 24, **characterized in that** the surface is provided with at least one spacer.

26. Method according to Claim 25, **characterized in that**, to remove the drop, the device, with the charged side facing downwards, is brought towards at least one depression in such a way that the drop touches at least one side wall of the depression and runs down it.

27. Method according to Claim 26, **characterized in that** the depression is a depression of a microtitre plate.

## Revendications

1. Dispositif pour créer des gouttelettes pendantes, le dispositif présentant une plaque avec au moins deux rehaussements constitués d'un matériau hydrophobe avec une surface limite supérieure rectangulaire sensiblement plane, sur laquelle à chaque fois entre 10 et 80 µl de milieu fluide peuvent être appliqués, la distance entre les rehaussements adjacents étant d'au moins 0,5 mm et les rehaussements présentant, sur la surface limite plane supérieure, au moins deux limitations à arêtes vives, et les dimensions du dispositif correspondant essentiellement à celles d'une plaque de microtitrage.

2. Dispositif selon la revendication 1, **caractérisé en ce que** les dimensions des rehaussements dans la première direction sont comprises entre 2 et 7 mm.

3. Dispositif selon l'une quelconque des revendications 1 ou 2, **caractérisé en ce que** les dimensions des rehaussements dans la deuxième direction sont comprises entre environ 3 et environ 9 mm.

4. Dispositif selon l'une quelconque des revendications 1 à 3, **caractérisé en ce que** les rehaussements sont des rehaussements étroits, étirés en longueur.

5. Dispositif selon l'une quelconque des revendications 1 à 4, **caractérisé en ce que** les rehaussements mesurent entre 1 et 5 mm de haut.

6. Dispositif selon l'une quelconque des revendications 1 à 5, **caractérisé en ce que** le nombre des rehaussements dans la direction longitudinale est d'au moins 18 et dans la direction transversale d'au moins 9.

7. Dispositif selon l'une quelconque des revendications 1 à 5, **caractérisé en ce que** le nombre des rehaussements dans la direction longitudinale est de 12 et dans la direction transversale de 8.

8. Dispositif selon l'une quelconque des revendications 1 à 7, **caractérisé en ce que** la distance entre deux rehaussements adjacents est comprise entre 0,5 et 4 mm.

9. Dispositif selon l'une quelconque des revendications 1 à 8, **caractérisé en ce que** le dispositif se compose au moins en partie d'au moins un matériau transparent.

10. Dispositif selon la revendication 9, **caractérisé en ce que** le matériau est du polystyrène et/ou du plexiglas.

11. Dispositif selon l'une quelconque des revendications 1 à 10, **caractérisé en ce que** les dimensions du dispositif dans la première direction sont comprises entre 100 et 150 mm et dans la deuxième direction entre 80 et 110 mm.

12. Dispositif selon l'une quelconque des revendications 1 à 11, **caractérisé en ce que** le dispositif présente une hauteur totale comprise entre 10 et 30 mm.

13. Dispositif selon l'une quelconque des revendications 1 à 12, **caractérisé en ce que** le dispositif a au moins une poignée.

14. Dispositif selon l'une quelconque des revendications 1 à 13, **caractérisé en ce que** le dispositif est réalisé de manière à pouvoir être déposé sur un bâti de support, le dispositif pouvant de préférence être déposé avec la surface à charger ou chargée vers le haut ou vers le bas.

15. Dispositif selon l'une quelconque des revendications 1 à 14, **caractérisé en ce que** le dispositif présente au moins un pied de support.

16. Dispositif selon l'une quelconque des revendications 1 à 15, **caractérisé en ce qu'**il est réalisé de manière empilable.

17. Procédé pour créer des conditions favorables de réaction et/ou de culture cellulaires, à l'exception des cellules souches embryonnaires humaines, **caractérisé par** l'utilisation d'un dispositif selon l'une quelconque des revendications précédentes, les rehaussements étant chargés d'un milieu fluide et des gouttelettes pendantes étant produites par rotation du dispositif.

18. Procédé selon la revendication 17, **caractérisé en ce que** les conditions de culture sont des conditions de prolifération et/ou de différenciation pour des cellules eucaryotes.

19. Procédé selon la revendication 17 ou la revendication 18, **caractérisé en ce que** le procédé est utilisé pour la culture de tissus et/ou la fabrication de tissus.

20. Procédé selon l'une quelconque des revendications 17 à 19, **caractérisé en ce que** les conditions de culture sont des conditions favorables et/ou restrictives et/ou destructives pour la culture de cellules tumorales et/ou de tissus tumoraux.

21. Procédé selon l'une quelconque des revendications 17 à 20, **caractérisé en ce que** les conditions de culture sont des conditions de croissance et/ou de différenciation pour les agglomérations cellulaires et/ou les tissus, pour influencer les processus angiogénétiques dans ces agglomérations cellulaires et/ou tissus.

22. Procédé selon la revendication 17, **caractérisé en ce que** les conditions de réaction sont des conditions d'analyse de cristallisation et/ou d'analyse de la structure radiographique.

23. Procédé d'utilisation de gouttelettes en tant qu'états de réaction, à l'exception des cellules souches embryonnaires humaines, **caractérisé en ce qu'**un milieu fluide est appliqué sur un dispositif selon l'une quelconque des revendications 1 à 16 et le dispositif est orienté avec le côté chargé vers le bas essentiellement horizontalement ou suivant un angle d'environ 90° ou moins par rapport à un subjectile.

24. Procédé selon la revendication 23, **caractérisé en ce que**, pour enlever la gouttelette, le dispositif est acheminé avec le côté chargé vers le bas et une surface essentiellement plane de telle sorte que la gouttelette se dépose sur la surface.

25. Procédé selon la revendication 24, **caractérisé en ce que** la surface est munie d'au moins un dispositif d'espacement.

26. Procédé selon la revendication 25, **caractérisé en ce que** pour enlever la gouttelette, le dispositif avec le côté chargé vers le bas, est rapproché d'au moins un renfoncement de telle sorte que la gouttelette vienne en contact avec au moins une paroi latérale du renfoncement et descende le long de celle-ci.

27. Procédé selon la revendication 26, **caractérisé en ce que** le renfoncement est un renfoncement de plaque de microtitrage.
